# EUROPEAN PATENT SPECIFICATION

(11) **EP 1 718 207 B1**
(45) Date of publication and mention of the grant of the patent: **20.10.2010**
(21) Application number: 05704869.6
(22) Date of filing: 12.01.2005
(51) Int. Cl.: A61B 5/15

(54) **LANCING DEVICE**
LANZETTENVORRICHTUNG
DISPOSITIF DE CREVAGE

(30) Priority: 15.01.2004 US 757776
(43) Date of publication of application: 08.11.2006
(73) Proprietor: NIPRO DIAGNOSTICS, INC., Fort Lauderale, FL 33309 (US)
(72) Inventor: BOEHM, David, K., Lauderhill, FL 33319 (US); CASTERLINE, Cameron, Scott, Pembroke Pines, FL 33029 (US)
(74) Representative: Molnia, David
(86) International application number: PCT/US2005/000014
(87) International publication number: WO 2005/070293

(56) References cited:
- US-A1- 2003 100 913
- US-B1- 6 530 937

## Description

### BACKGROUND

### 1. Field of the Invention

The present invention relates to lancing devices and, more particularly, to apparatuses and methods for lancing and assembling lancing devices.

### 2. Description of Related Art

Many people have a need to regularly monitor their blood. Diabetics, for example, need to regularly monitor their blood glucose levels. Lancing devices, generally, offer a relatively pain-free, quick, and sterile means to prick a person's skin to obtain a drop of blood. The blood may then be collected and/or tested, in a blood glucose meter, for example.

To obtain a drop of blood, a spring-loaded lancing device can be placed in contact with a person's skin. The loaded spring can then be released, and the lancing device can propel a needle-tipped lancet through a hole in a nozzle of the lancing device into the skin. The needle can then penetrate the skin for only a fraction of a second before being retracted back into the lancing device.

The depth that the needle penetrates the skin can be considered the lancing depth. The lancing depth can then generally be equal to the furthest length that the needle extends from the nozzle of the lancing device as the needle penetrates the skin.

The lancing depth can be controlled, for example, by using a particular length needle. Another, more convenient, way to control the lancing depth is by rotating the nozzle of the lancing device to adjust the relative distance between the nozzle and the tip of the lancet's needle. For instance, for a shallower lancing depth, the nozzle can be moved closer to the needle tip at the needle tip's furthest distance from the nozzle. For a deeper lancing depth, the nozzle can be moved further away from the needle tip at the needle tip's furthest distance from the nozzle. Examples of depth control is present in the prior art. US patent number 6,530,937 B1 discloses an adjustable tip for a lancet device and method where the tip for a lancet device may not extend beyond an extend stop element, a slot is disposed in one of a sidewall portion and at least one following element extending into the slot. A stop cap has ratchet teeth which engage internal spline of a front cap and moves with respect to inner sliding surface of the front cap. A set depth of penetration of the needle by moving a the at least one following element disposing the distal end of the lancet device against a surface of skin and triggering the firming mechanism to cause the needle to penetrate the surface of the skin to the set depth. In a second example, US patent application number 2003/0100913 A1 discloses adjusting depth of lancing device cap. In this application, there is a screw structure in the lancet cover along the axes to control the exposed lancet length, and when rotating the adjustable cover, the rotary position structure makes the adjustable cover move intermittently, where the user hears a 'click' to indicate movement.

A lancing depth that can be conveniently adjusted has many advantages. Different fingers can have different skin thicknesses, for example. Different areas of the body might also have different skin thicknesses. Adjusting a lancing device to the appropriate lancing depth can therefore minimize the pain of lancing too deeply into the skin and can help ensure that the lancet needle penetrates the skin a sufficient depth to obtain a drop of blood.

Lancing devices, generally, can be beneficial for many reasons. For example, lancing devices offer a less painful way to prick skin to obtain a drop of blood. Pain is minimized because the lancet's needle is in the skin for only a fraction of a second. Lancing devices can also minimize the emotional trauma of pricking skin. When using a lancing device one can simply, for example, press a button to lance one's skin, instead of somehow holding a needle and directly pushing the needle into the skin. Lancing devices can also hide the lancet needle within an enclosure, thereby allowing the person being stuck to avoid looking at the needle. In a clinical setting, lancing devices can also allow a nurse or other caregiver to quickly and relatively painlessly prick a patient's skin to obtain a drop of blood.

Accordingly, there is a need to provide convenient and reliable lancing devices, methods of lancing, and methods of assembling lancing devices.

### SUMMARY OF THE INVENTION

Exemplary embodiments include a simple to operate handheld device with a "pen like" ergonomic styling. One embodiment has five adjustable lancing depth settings. The embodiments can be used for normal fingertip lancing or for alternate site testing, and can use standard (universal), "off the shelf lancets. The lancing device can also be constructed of various injection molded plastic parts that can "snap-fit" or "pressure-fit" together.

In a first embodiment, the present invention provides an adjustable - nozzle assembly through which a lancet can be propelled by a lancing device into a lancing surface. The adjustable nozzle assembly includes an interior nozzle comprising a ramped groove and a lancet wall; a collar comprising a collar pin that engages the ramped groove and slides relative to the ramped groove, the collar being adapted to rotate relative to the interior nozzle; and an exterior nozzle comprising a contact surface that extends beyond the lancet wall of the interior nozzle to contact the lancing surface, the exterior nozzle engaging the collar and being adapted to rotate relative to the interior nozzle. The ramped groove is sloped such that as the exterior nozzle rotates relative to the interior nozzle, the distance that the contact surface extends beyond the lancet wall changes by an amount that corresponds to the slope of the ramped groove. In this and other embodiments, the collar can further comprise a detent, and the interior nozzle can further comprise a plurality of adjustment notches that can engage the detent.

In a second embodiment, the present invention provides a rearward body assembly of a lancing device that can propel a lancet into a lancing surface. The rearward body assembly includes a lancet holder comprising one or more retaining features and one or more spring surfaces; an interior tube comprising an open end and a slotted end through which the one or more retaining features extend, the interior tube being adapted to slidably engage the lancet holder; an internal compression spring comprising a first end and a second end, the first end of the internal compression spring being adapted to act on the slotted end of the interior tube and the second end of the internal compression spring being adapted to act on the one or more spring surfaces of the lancet holder; a retainer comprising a slotted surface through which the one or more retaining features extend; a rearward body, the rearward body engaging the retainer; and an external compression spring comprising a first end and a second end, the first end comprising a reduced coil diameter that engages the one or more retaining features of the lancet holder, the first end of the external compression spring being adapted to act on the lancet holder, and the second end of the external compression spring being adapted to act on the slotted-surface of the retainer. Longitudinal movement of the rearward body away from the interior tube compresses the interior compression spring.

In a third embodiment, the present invention provides that the rearward body assembly of the second embodiment can releasably engage the adjustable nozzle assembly, of the first embodiment.

In a fourth embodiment, the present invention provides a method of assembling an adjustable nozzle assembly of a lancing device. The method of the fourth embodiment includes providing an interior nozzle comprising an assembly groove in communication with a ramped groove, the assembly groove being separated from the ramped groove by a raised boss; providing a collar with a collar pin; and attaching the collar to the interior nozzle by sliding the collar pin in the assembly groove, over the raised boss, and into the ramped groove. In this and other embodiments, the collar can further comprise a detent, and the interior nozzle can further comprise a plurality of adjustment notches that can engage the detent.

In a fifth embodiment, the present invention provides a method of adjusting a lancing depth of a nozzle assembly in a lancing device, the nozzle assembly comprising an exterior nozzle, an interior nozzle comprising a ramped groove, and a collar engaging the- exterior nozzle and comprising a collar pin that can slidably engage the ramped groove. The method includes rotating the exterior nozzle and the collar relative to the interior nozzle to slide the collar pin in the ramped groove of the interior nozzle to adjust the lancing depth.

Other embodiments are possible as well, and a variety of alternatives will become apparent to those skilled in the art upon review of the following detailed description.

### BRIEF DESCRIPTION OF THE DRAWINGS

Exemplary embodiments of the present invention are described herein with reference to the drawings, in which:

Figure 1 is a perspective view of an exemplary lancing device;

Figure 2 is a perspective view of an exemplary lancet;

Figure 3 is an exploded perspective view of several components of the lancing device, including a finger cover, a trigger, and an interior tube;

Figure 4 is a cross-sectional perspective view of several components of the lancing device, including the components of Figure 3 and a lancet holder and an internal spring;

Figure 4A is a perspective view of a detail of the finger cover engaging the interior tube;

Figure 5 is a perspective view of the components of Figure 4 engaging a retainer and an external spring;

Figure 6 is an exploded perspective view of the components of Figure 5 engaging a rearward body;

Figure 7 is a perspective view of a rearward body assembly, including the finger cover, the trigger, the interior tube, and the lancet holder;

Figure 8 is an exploded perspective view of an exterior nozzle and a collar of the lancing device;

Figure 8A is a perspective view of a detail of the exterior nozzle and the collar;

Figure 9 is an exploded perspective view of the components of Figure 8 and an interior nozzle;

Figure 9A is a perspective view of an alternative collar embodiment;

Figure 10 is an exploded perspective view of the components of Figure 8 and the interior nozzle;

Figure 11 is a perspective view of the nozzle assembly, including the exterior nozzle and the interior nozzle;

Figure 12 is a perspective view of the nozzle assembly and the rearward body assembly engaging the lancet;

Figure 13 is a perspective view of an exemplary exterior nozzle configuration;

Figure 14 is a perspective view of an exemplary exterior nozzle configuration; and

Figure 15 is a perspective view of an exemplary exterior nozzle configuration.

### DESCRIPTION OF THE EMBODIMENTS

### 1. Exemplary Lancing Device Configures

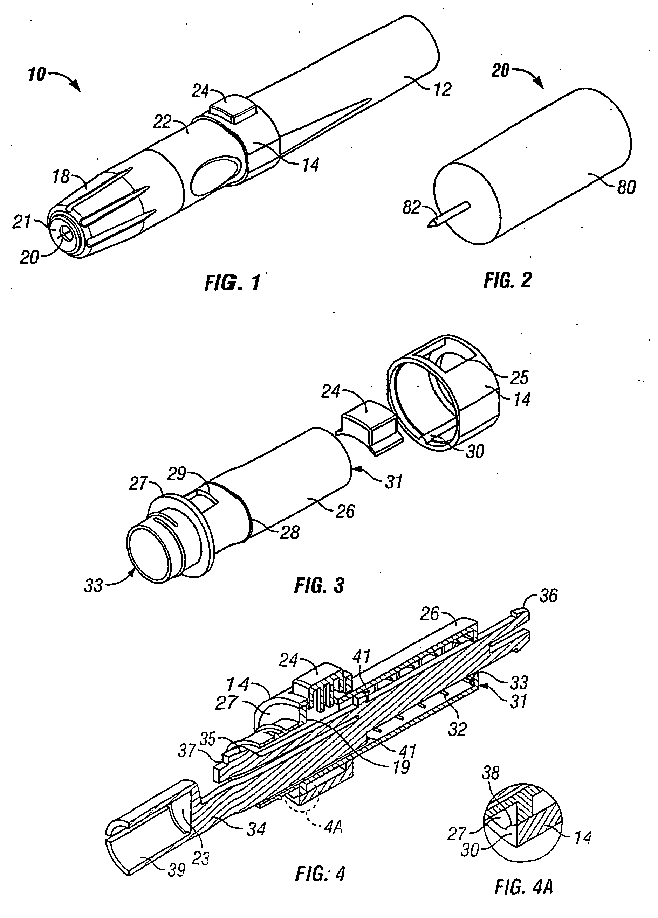

Figure 1 depicts an exemplary lancing device 10. The lancing device 10 can include a rearward body 12, a finger cover 14, an exterior nozzle 1 8 with a contact surface 21, an interior nozzle 22, and a trigger 24. The components of the lancing device 10 can be assembled to spring load and propel a lancet 20. Upon being propelled, the tip of the lancet 20 can extend beyond the contact surface 2 1 a particular, adjustable lancing depth. In some embodiments, the lancet 20 is removable or replaceable. For instance, in many applications it is advantageous to have a disposable, single-use lancet 20.

Figure 2 depicts an exemplary lancet 20 for use in the lancing device 10.' As shown in Figure 2, the lancet 20 can comprise a needle section 82 and a barrel section 80. The lengths and diameters of each of the needle section 82 and the barrel section 80 can vary. Although the barrel section 80 is shown as smooth, the barrel section 80 can also be contoured.

Figure 3 depicts an exploded view of several components of the lancing device 10, in particular, an interior tube 26, the finger cover 14, and the trigger 24. As shown in Figure 3, the interior tube 26 has a distal end 31 and a proximal end 33, and can include a snap ring 28, a raised ring 27, and an interior tube opening 29. The finger cover 14 can comprise a finger cover alignment feature 30, which might be an elongated protrusion on the inner surface of the interior tube 26, and a trigger opening 25. Once assembled, the trigger 24 can extend outwardly though the trigger opening 25 and can extend inwardly through the interior tube opening 29.

Figure 4 depicts a cross-sectional perspective view of several components of the lancing device 10, including the components of Figure 3, an internal spring 32, and a lancet holder 34. In an exemplary embodiment, the internal spring 32 is a compression spring that can act against the distal end 31 of the interior tube 26 and against the lancet holder 34 to propel the lancet 20.

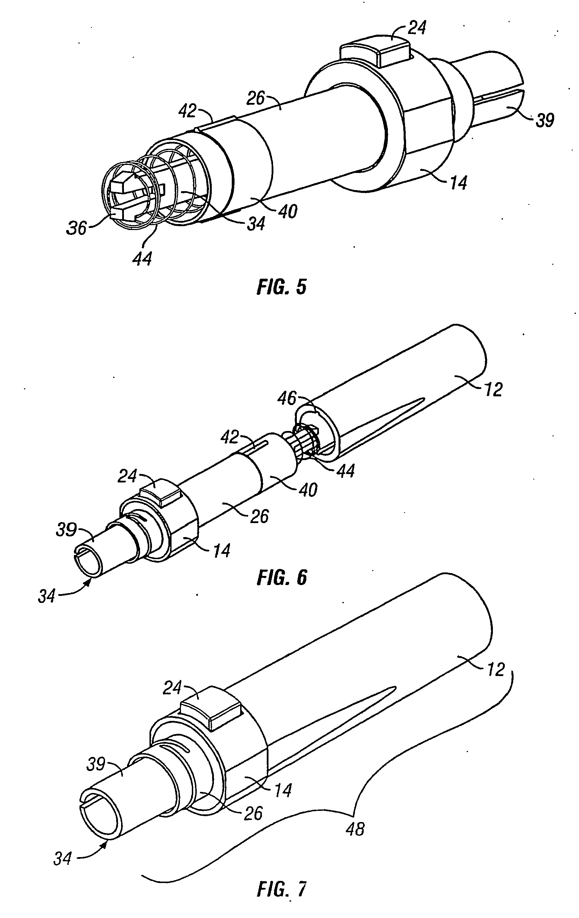

In an exemplary embodiment, the lancet holder 34 includes a plurality of components and serves a plurality of functions. The lancet holder 34 can comprise a barrel holder 39 and a seat 23, both of which can hold and help propel the lancet 20. The lancet holder 34 can also comprise one or more internal spring surfaces 41 against which the internal spring 32 can act to propel the lancet 20. Further, the lancet holder 34 can comprise one or more retaining features 36 that can extend through a slot 33 of the interior tube 26 and can help retain an external spring 44 and a retainer 40 (both shown in Figure 5). In addition, the lancet holder can also comprise a cantilevered trigger extension 35, which can be biased such that it can spring load the trigger 24.

To spring load the trigger 24 and prepare the lancing device 10 for use, the trigger extension 35 can be aligned with the interior tube opening 29 by compressing the internal spring. 32. Once sufficiently aligned, the bias of the trigger extension 35 can cause the trigger extension 35 to extend toward the opening 29 and engage the trigger 24. A trigger extension notch 37 on the trigger extension 35 can then engage a corner 19 of the interior tube 26. The trigger extension 35 can thereby oppose longitudinal movement of the lancet holder 34 and can oppose the longitudinal force caused by the compressed internal spring 32.

There is thus a "loaded" position of the lancing device 10 in which the internal spring 32 is compressed and the engagement of the notch 37 with the interior tube 26 opposes the release of the compressed internal spring 32. In the loaded position, the trigger extension 35 engages the trigger 24 such that, in an exemplary embodiment, the trigger is somewhat raised from the surface of the finger cover 14 and offers some resistance to movement. To propel the lancet 20, a user can actuate the trigger 24 to release the engagement of the notch 37 with the interior tube 24 and to thereby release the internal spring 32. The internal spring 32 can then move the lancet holder 34 longitudinally to propel the lancet 20.

In assembly, both the finger cover alignment feature 30 and the snap ring 28 can help align the finger cover 14 with other components of the lancing device 10. As shown in Figure 4A, the finger cover alignment feature 30 can help align the finger cover 14 along the periphery of the interior tube 26 by engaging a notch 38 in the raised ring 27. In an exemplary embodiment, the snap ring 28 can help align the finger cover 14 along the longitudinal axis of the interior tube 26 by engaging an edge of the finger cover 14.

Figure 5 depicts the components of Figure 4 engaging a retainer 40 and an external spring 44. The external spring 44 can engage the retaining features 36 of the lancet holder 34 and an end of the retainer 40. In an exemplary embodiment, the diameter of the external spring 44 is reduced near where the external spring 44 engages the retaining features 36; and, in an exemplary embodiment, the retaining features 36 can include an aggressive undercut to facilitate engagement with the external spring 44. Advantageously, the reduced external spring diameter allows the external spring 44 to act directly on the lancet holder. The reduced spring diameter can thus eliminate the need for an additional component for the external spring 44 to act on, such as a slotted disc connected to the retaining features 36 of the lancet holder 34. In an exemplary embodiment, the retainer 40 can include one or more retainer alignment features 42.

Figure 6 depicts an exploded view of the components of Figure 5 engaging the rearward body 12. In an exemplary embodiment, the rearward body 12 can include one or more grooves 46. The grooves 46 can engage the retainer alignment features 42 and can thereby help align the rearward body 12 along the circumference of the lancing device 10. Advantageously; after the rearward body 12 is assembled with the retainer 40, the grooves 46 and the retainer alignment features 42 can also oppose rotation of the rearward body along its longitudinal axis. The rearward body 12 might also include a snap ring feature (not shown) that can help align the rearward body 12 along the longitudinal axis of the lancing device 10 and that can help ensure that the rearward body 12 is fully engaged with the lancing device 10..

Figure 7 depicts a rearward body assembly 48, which includes the rearward body 12 assembled with the finger cover 14, the trigger 24, the interior tube 26, and the lancet holder 34. A user can "load" the rearward body assembly 48 by longitudinally pulling the rearward body 12 away from the finger cover 14. Because the rearward body 12 is coupled to the retainer 40, and because the retainer 40 is coupled to the lancet holder 34 (via the external spring 44), the movement of the rearward body 12 will compress the internal spring 32 and will longitudinally move the lancet holder 34 relative to the interior tube 26. Once the trigger extension 35 is sufficiently aligned with the interior tube opening 29, the trigger extension 35 can engage the trigger 24, and the notch 37 can engage the interior tube 26 to oppose the release of the compressed spring 32. The user can then release the rearward body 12 and the external spring 44 will return the rearward body 12 to the position depicted in Figure 7. Once the rearward body assembly 48 is loaded, the user can then actuate the trigger 24 to release the lancet holder 34.

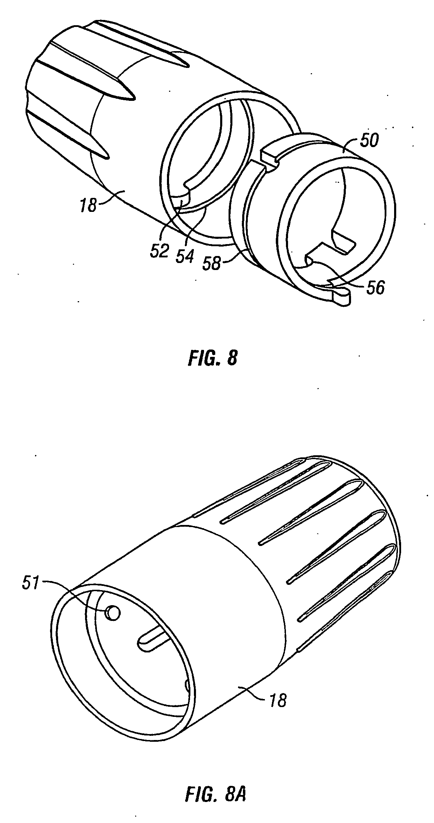

Figure 8 depicts an exploded view of the exterior nozzle 18 and a collar 50. In an exemplary embodiment, the collar 50 comprises a retaining groove 58 and one or more collar alignment features 56, and the exterior nozzle 18 comprises a retaining ring 54 and one or more exterior nozzle alignment features 52. The collar 50 can then be inserted into the exterior nozzle 18, and the retaining groove 58 can engage the retaining ring 54 .to help align and retain the collar 50 along the longitudinal axis of the exterior nozzle 18. Further, the collar alignment features 56 can engage the exterior nozzle alignment features 52 to help align and retain the collar 50 along the interior circumference of the exterior nozzle 18.

The interior surface of the collar 50 can also comprise a collar pin 51, which is depicted in Figure 8A. As shown in Figure 8A, the collar pin 51 can extend from the interior surface of the collar 50.

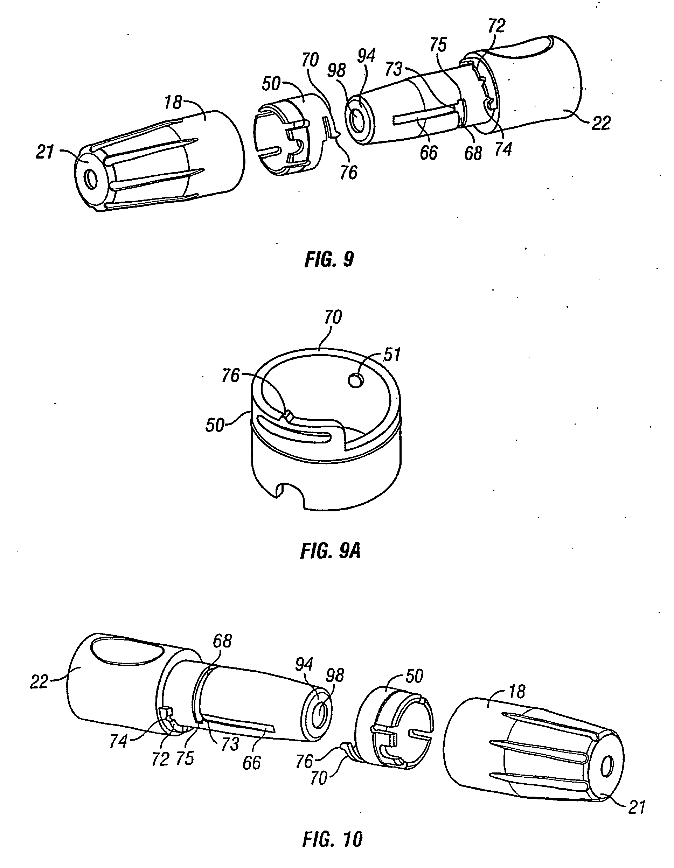

Figures 9 and 10 depict exploded views (from different perspectives) of the components of Figure 8 and the interior nozzle 22. As shown in Figures 9 and 10, the interior nozzle 22 can comprise an assembly groove 66 and a ramped groove 68. The collar 50 can then engage the interior nozzle 22 via the collar pin 51 (shown in Figure 8A), which can engage the assembly groove 66 and the ramped groove 68 of the interior nozzle 22.

In an exemplary embodiment, to assemble the collar 50 and the interior nozzle 22, one can slide the collar pin 51 through the assembly groove 66-to the ramped groove 68. Near where the assembly groove 66 meets the ramped groove 68, the interior nozzle 22 can comprise a raised boss 73. In assembly, the collar pin 51 can be slid in the assembly groove 66 and can then snap over the raised boss 73 into the ramped groove 68.

Once assembled, the raised boss 73 creates a tortuous path that can make disassembly of the collar 50 (and hence the exterior nozzle 18) from the interior nozzle 22 difficult. The ramped groove 68 can retain the collar pin 51 and can allow the collar to rotate about the periphery of the interior nozzle 22. The interior nozzle 22 can also comprise an over rotation groove 75 that can retain the collar pin 51 when the collar is fully rotated and can help prevent disassembly of the collar 50 (and hence the exterior nozzle 18) from the interior nozzle 22 when the collar 50 and the exterior nozzle 18 are fully rotated.

As shown in Figures 9 and 10, the interior nozzle 22 can also comprise an interior nozzle mating ramp 72, which can include a plurality of adjustment notches 74 (in a preferred embodiment, for example, there are five adjustment notches 74). The collar 50, in turn, can comprise a collar mating ramp 70, which can include a detent 76. The detent 76 can form a biased, flexible portion of the collar mating ramp 70, to allow the detent to engage and disengage the adjustment notches 74. In an exemplary embodiment, the detent 76 forms a. cantilevered portion of the collar mating ramp 70. In an alternative embodiment depicted in Figure 9A, the detent 76 forms a slotted portion of the collar mating ramp 70. Other examples are possible as well.

The interior nozzle mating ramp 72, the collar mating ramp 70, and the ramped groove 68, can each be sloped relative to a plane perpendicular to the longitudinal axis of the lancing device 10. Further, in an exemplary embodiment, the slopes of each of the interior nozzle mating ramp 72, the collar mating ramp 70, and the ramped groove 68 should be approximately the same or similar. Once assembled, the collar pin 51 can then slide in the ramped groove 68 and the collar mating ramp 70 and the detent 76 can rotate along the interior nozzle mating ramp 72.

As shown in Figures 9 and 10, the interior nozzle 22 can comprise a lancet wall 94 with a lancet wall opening 98. When a lancet 20 is propelled by the lancing device 10, the lancet wall opening 98 can provide an opening through which the needle section 82 can extend beyond the lancet wall 94 toward the surface to be lanced. The lancet wall 94, in turn, can engage the barrel section 80 of the lancet 20 and can act to stop the movement of a propelled lancet 20.

As discussed above, in an assembled lancing device 10, the exterior nozzle 18 engages the collar 50. Thus, the collar pin 51, the collar 50, and the exterior nozzle 18 rotate together about the periphery of the interior nozzle 22. By sliding in the ramped groove 68,-the collar pin 51 can act to vary the distance that the contact surface 21 of the exterior nozzle 18 extends from the lancet wall 94, and, hence, can act to vary the distance that the lancet 20 extends from the contact surface 21. Thus, by rotating the exterior nozzle 18 relative to other components of the lancing device 10, one can vary the lancing depth according to the slope of the ramped groove 68.

In an exemplary embodiment, particular lancing depth positions can be defined by the adjustment notches 74 along the slope of the interior nozzle mating .ramp 72. Then as the exterior nozzle 18 is rotated, the detent 76 of the collar mating ramp 70 can engage the various adjustment notches 74. The engagement between the detent 76 and each adjustment notch 74 can help define a particular lancing depth for the lancet 20. An indication of the location and the corresponding, relative lancing depth represented by each of the adjustment notches 74 can be marked on the outside surface of the lancing device 10.

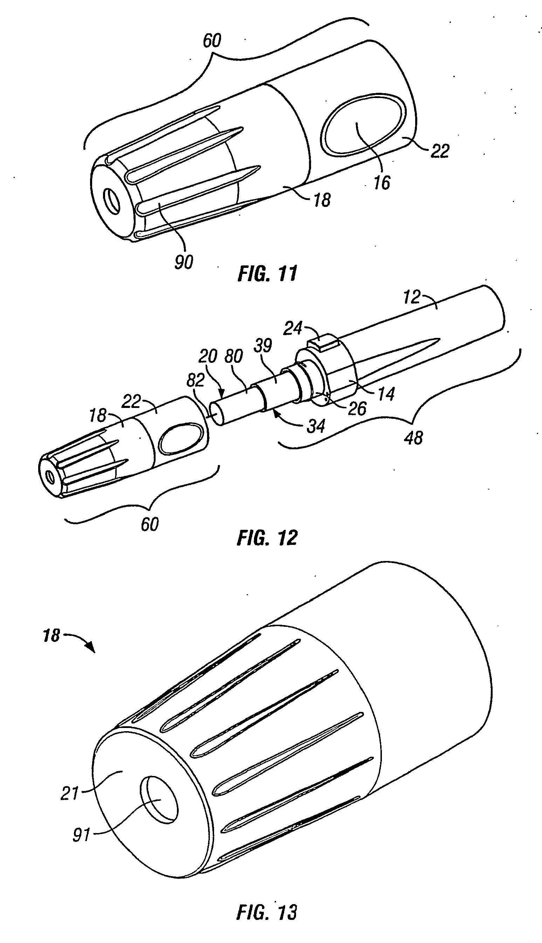

Figure 11 depicts a nozzle assembly 60, which includes the exterior nozzle 18, the collar 50 (shown in Figures 9 and 10), and the interior nozzle 22. As discussed above, a user can adjust the lancing depth of the lancet 20 by rotating the exterior nozzle 18 relative to the interior nozzle 22. To facilitate the rotation of the exterior nozzle 18, the exterior nozzle 18 can comprise a plurality of grip features 90, and the interior nozzle 22 can comprise one or more finger grips 16.

One can then hold the interior nozzle 22 at the finger grips 16 and rotate the exterior nozzle 18 using the grip features 90. In an exemplary embodiment, as the exterior nozzle 18 is rotated; the detent 76 can "click" into the various adjustment notches 76 (shown in Figures 9 and 10) on the interior nozzle 22.

Figure 12 depicts a perspective view of the nozzle assembly 60 arid the rearward body assembly 48 engaging the lancet 20. As shown in Figure 12, the barrel holder 39 of the lancet holder 34 can engage the barrel section 80 of the lancet 20. The lancet 20 can then rest in the seat 23 (shown in Figure 4) of the lancet holder 34. In an exemplary embodiment, the interior diameter of the barrel holder, 39 can approximately equal the outside diameter of the barrel section 80.

As also shown in Figure 12, in an exemplary embodiment, the nozzle assembly 60 can be releasably coupled to the rearward body assembly 48 via a threaded connection, for example. Other methods of attachment, such as a snap-fit, quick-release, or sliding coupling, are possible as well. In any case, the advantages of such a releasable coupling include allowing a user to disengage the nozzle assembly 60 from the rearward body assembly 48 to insert or remove a lancet 20 from the lancet holder 34.

Figures 13, 14, and 15 depict exemplary exterior nozzle 18 configurations. Each exterior nozzle 18 includes the contact surface 21, which can contact the surface that the lancet 20 will lance.. The contact surface 21, in turn, will comprise a lancet opening 91, through which the needle section 82 of a lancet 20 can extend to lance a surface, such as a person's skin. In general, it is advantageous for the lancing device 10 to have a contact surface 21 that is shaped to encourage the flow of blood to the point where the lancet 20 lances the skin.

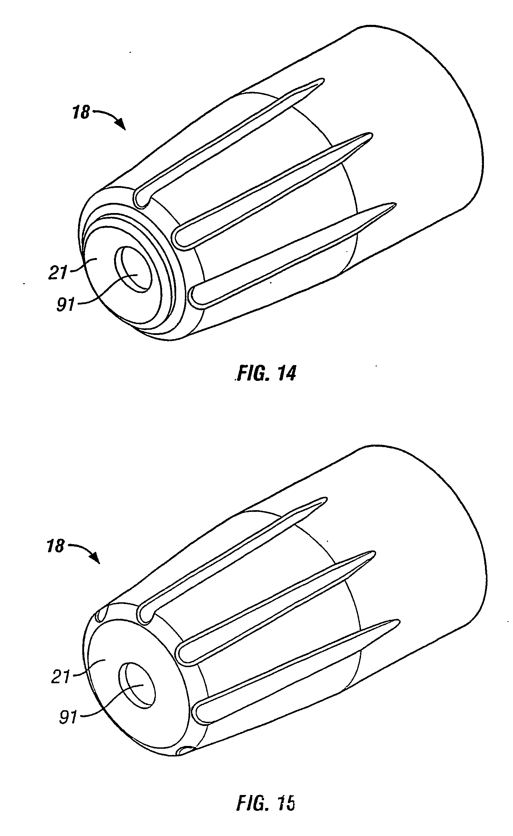

The exterior nozzles depicted in Figures 13, 14, and 15 differ in that the contact surface 21 of each exterior nozzle 18 has different features. Figure 13 depicts the exterior nozzle 18 with a concave contact surface 21. Although the contact surface 21 shown in Figure 13 is smooth, the surface 21 can also have a contoured topography. Figure 14 depicts the exterior nozzle 18 with a raised-ring, contoured contact surface 21. And Figure 15 depicts the exterior nozzle 18 with a planar contact surface 21. Other examples, such as segmented contact surfaces, for example, are possible as well.

### 2. Exemplary Operation

With reference to the above discussed figures, operation of the lancing device 10 can include several steps. These steps might include insertion of a lancet 20 into the lancing device 10, selection of a lancing depth, spring loading the lancing device 10, and actuating the trigger 24 to lance a surface, such as a person's skin.

In particular, to insert a lancet 20 into the lancing device 10, a user can disengage the nozzle assembly 60 from the rearward body assembly 48 to expose the barrel holder 39. of the lancet holder 34. The user can then insert the barrel section 80 of the lancet 20 into the barrel holder 39 until the lancet 20 rests against the seat 23 of the lancet holder 34. As necessary, the user can remove any protective or safety cover on the lancet 20 to expose the needle section 82 of the lancet 20. The user can then re-engage the nozzle assembly 60 with the rearward body assembly 48.

Next the user can select a particular lancing depth of the lancet by rotating the exterior nozzle 18. The exterior nozzle 18 can be marked with identifications of available lancing depths that correspond with the adjustment notches 74. As the user rotates the exterior nozzle 18, the detent 76 can click into the adjustment notches 74, indicating to the user that a particular lancing depth has been selected.

The user can then spring load the lancing device 10 by pulling the rearward body 12 along the longitudinal axis of the lancing device 10. To hold the lancing device while pulling the rearward body 12, the user can grip another component, such as the finger cover 14 or the interior nozzle 22. In an exemplary embodiment, once the user has pulled the rearward body 12 a sufficient amount, the user can see and/or hear the trigger extension 35 engaging the trigger 24 and/or the trigger extension notch 37 engaging the interior tube 26. The user can then release the rearward body 12, which will return to its original position via the external spring 44 acting on the retainer 40.

To use the lancing device 10, the user can place the contact surface 21 of the external nozzle 18 on the surface to be lanced, such as a. person's skin. The user can then actuate the trigger 24 and the internal spring 32 will propel the lancet holder 34 and, hence, the lancet 20, toward the lancing surface. The needle section 82 will also be propelled through the lancet wall opening 98, through the lancet opening 91, and into the lancing surface, up to the lancing depth. Blood can then be collected from the lanced surface and tested.

## Claims

1. An adjustable nozzle assembly (60) through which a lancet (20) can be propelled by a lancing device (10) into a lancing surface (21), the adjustable nozzle assembly, comprising:
an interior nozzle (22) comprising a ramped groove (68) and a lancet wall (94); a collar (50) comprising a collar pin (51) that engages the ramped groove and slides relative to the ramped groove, the collar being adapted to rotate relative to the interior nozzle; and an exterior nozzle (18) comprising a contact surface (21) that extends beyond the lancet wall (94) of the interior nozzle to contact the lancing surface, the exterior nozzle engaging the collar and being adapted to rotate relative to the interior nozzle; and wherein the ramped groove is sloped such that as the exterior nozzle rotates relative to the interior nozzle, the distance that the contact surface extends beyond the lancet wall changes, by an amount that corresponds to the slope of the ramped groove.

2. The nozzle assembly of claim 1, wherein the collar further comprises a cantilevered detent (76) and wherein the interior nozzle further comprises a plurality of adjustment notches (74) that can engage the cantilevered detent.

3. The nozzle assembly of claim 1, wherein:
the collar further comprises a sloped collar ramp (70), the sloped collar ramp comprising a detent (76); and
the interior nozzle further comprises a sloped interior nozzle ramp (72), the sloped interior nozzle ramp comprising a plurality of adjustment notches (74) that can engage the detent; and
wherein the slope of the collar ramp, the slope of the interior nozzle ramp, and the slope of the ramped groove are approximately equal.

4. The nozzle assembly of claim 3, wherein the detent forms a cantilevered portion of the collar ramp.

5. The nozzle assembly of claim 3, wherein the detent forms a slotted portion of the collar ramp.

6. The nozzle assembly of claim 1, wherein the interior nozzle further comprises an assembly groove (66), one end of the assembly groove being in proximity to one end of the ramped groove, the assembly groove comprising a raised boss (73) that can oppose the collar pin sliding from the ramped groove to the assembly groove.

7. The nozzle assembly of claim 1, wherein the collar further comprises one or more collar alignment features, and the exterior nozzle further comprises one or more exterior nozzle alignment features (52) that can engage the one or more collar alignment features (56).

8. The nozzle assembly of claim 1, wherein the contact surface is concave.

9. The nozzle assembly of claim 1, wherein the ramped groove comprises an over rotation groove.

10. A method of assembling the adjustable nozzle assembly (60) of claim 1 of a lancing device (10), the method comprising:
providing an interior nozzle (22) comprising an assembly groove (66) in communication with a ramped groove (68), the assembly groove being separated from the ramped groove by a raised boss (73);
providing a collar (50) with a collar pin (51); and
attaching the collar to the interior nozzle by sliding the collar pin in the assembly groove, over the raised boss, and into the ramped groove.

11. The method of claim 10, wherein the collar further comprises a cantilevered detent (76) and wherein the interior nozzle further comprises a plurality of adjustment notches (74) that can engage the cantilevered detent.

12. The method of claim 10, wherein:
the collar further comprises a sloped collar ramp(70), the sloped collar ramp comprising a cantilevered detent (76); and
the interior nozzle further comprises a sloped interior nozzle ramp (72), the sloped interior nozzle ramp comprising a plurality of adjustment notches (74) that can engage the cantilevered detent; and
wherein the slope of the collar ramp, the slope of the interior nozzle ramp, and the slope of the ramped groove are approximately equal.

13. The lancing device of claim 12, wherein the detent forms a cantilevered portion of the collar ramp.

14. The lancing device of claim 12, wherein the detent forms a slotted portion of the collar ramp.

15. The method of claim 10, wherein the raised boss can oppose the collar pin sliding from the ramped groove to the assembly groove.

16. The method of claim 10, wherein the ramped groove comprises an over rotation groove (75).

17. The method of claim 10, wherein the collar further comprises one or more collar alignment features, the method further comprising:
providing an exterior nozzle (18) comprising one or more exterior nozzle alignment features (52) that can engage the one or more collar alignment features (56);
aligning the one or more collar alignment features (56) with the one or more exterior nozzle alignment features(52); and engaging the exterior nozzle (18) to the collar (50).

## Patentansprüche

1. Regelbare Düsenanordnung (60) über die eine Lanzette (20) durch eine Stechvorrichtung (10) in eine Einstichfläche (21) hineingetrieben werden kann, wobei die regelbare Düsenanordnung Folgendes umfasst: eine innere Düse (22), umfassend eine schräge Nut (68) und eine Lanzettenwand (94), eine Manschette (50) umfassend einen Manschettenstift (51), der in die schräge Nut eingreift und bezüglich der schrägen Nut gleitet, wobei die Manschette so angepasst ist, dass sie bezüglich der inneren Düse rotiert, und eine äußere Düse (18), die eine Kontaktfläche (21) umfasst, die sich über die Lanzettenwand (94) der inneren Düse hinaus erstreckt, so dass sie die Einstichfläche berührt, wobei die äußere Düse in die Manschette eingreift und so angepasst ist, dass sie bezüglich der inneren Düse rotiert, und wobei die schräge Nut so geneigt ist, dass sich, wenn die äußere Düse bezüglich der inneren Düse rotiert, der Abstand, über den sich die Kontaktfläche über die Lanzettenwand hinaus erstreckt um einen Betrag ändert, der der Neigung der schrägen Nut entspricht.

2. Düsenanordnung nach Anspruch 1, wobei die Manschette außerdem ein vorkragendes Sperrelement (76) aufweist und wobei die innere Düse außerdem eine Vielzahl von Einstellkerben (74) umfasst, die in das vorkragende Sperrelement eingreifen können.

3. Düsenanordnung nach Anspruch 1, wobei:
die Manschette außerdem eine geneigte Manschettenrampe (70) aufweist, wobei die geneigte Manschettenrampe ein Sperrelement (76) aufweist, und
die innere Düse außerdem eine geneigte innere Düsenrampe (72) aufweist, wobei die geneigte innere Düsenrampe eine Vielzahl von Einstellkerben (74) aufweist, die in das Sperrelement eingreifen können, und
wobei die Neigung der Manschettenrampe, die Neigung der inneren Düsenrampe und die Neigung der schrägen Nut ungefähr gleich sind.

4. Düsenanordnung nach Anspruch 3, wobei das Sperrelement einen vorkragenden Teil der Manschettenrampe bildet.

5. Düsenanordnung nach Anspruch 3, wobei das Sperrelement einen schlitzförmigen Teil der Manschettenrampe bildet.

6. Düsenanordnung nach Anspruch 1, wobei die innere Düse außerdem eine Montagenut (66) umfasst, wobei ein Ende der Montagenut nahe des einen Endes der schrägen Nut angeordnet ist, wobei die Montagenut einen erhöhten Anguss (73) aufweist, der dem Gleiten des Manschettenstifts von der schrägen Nut auf die Montagenut entgegenwirken kann.

7. Düsenanordnung nach Anspruch 1, wobei die Manschette außerdem ein oder mehrere Manschettenausrichtungs-Merkmale umfasst und die äußere Düse außerdem ein oder mehrere äußere Düsenausrichtungs-Merkmale (52) umfasst, die in das eine oder die mehreren Manschettenausrichtungs-Merkmale (56) eingreifen können.

8. Düsenanordnung nach Anspruch 1, wobei die Kontaktfläche konkav ist.

9. Düsenanordnung nach Anspruch 1, wobei die schräge Nut eine Überdreh-Nut aufweist.

10. Verfahren zur Montage der regelbaren Düsenanordnung (60) nach Anspruch 1 einer Stechvorrichtung (10), wobei das Verfahren Folgendes umfasst:
Bereitstellen einer inneren Düse (22), die eine Montagenut (66) in Verbindung mit einer schrägen Nut (68) umfasst, wobei die Montagenut durch einen erhöhten Anguss (73) von der schrägen Nut getrennt ist,
Bereitstellen einer Manschette (50) mit einem Manschettenstift (51) und
Befestigen der Manschette an der inneren Düse durch Schieben des Manschettenstifts in die Montagenut, über den erhöhten Anguss und in die schräge Nut.

11. Verfahren nach Anspruch 10, wobei die Manschette außerdem ein vorkragendes Sperrelement (76) umfasst und wobei die innere Düse außerdem eine Vielzahl von Einstellkerben (74) umfasst, die in das vorkragende Sperrelement eingreifen können.

12. Verfahren nach Anspruch 10, wobei:
die Manschette außerdem eine geneigte Manschettenrampe (70) umfasst, wobei die geneigte Manschettenrampe ein vorkragendes Sperrelement (76) umfasst, und
die innere Düse außerdem eine geneigte innere Düsenrampe (72) umfasst, wobei die geneigte innere Düsenrampe eine Vielzahl von Einstellkerben (74) umfasst, die in das vorkragende Sperrelement eingreifen können, und
wobei die Neigung der Manschettenrampe, die Neigung der inneren Düsenrampe und die Neigung der schrägen Nut ungefähr gleich sind.

13. Stechvorrichtung nach Anspruch 12, wobei das Sperrelement einen vorkragenden Teil der Manschettenrampe bildet.

14. Stechvorrichtung nach Anspruch 12, wobei das Sperrelement einen schlitzförmigen Teil der Manschettenrampe bildet.

15. Verfahren nach Anspruch 10, wobei der erhöhte Anguss dem Gleiten des Manschettenstifts von der schrägen Nut auf die Montagenut entgegenwirken kann.

16. Verfahren nach Anspruch 10, wobei die schräge Nut eine Überdreh-Nut (75) umfasst.

17. Verfahren nach Anspruch 10, wobei die Manschette außerdem ein oder mehrere Manschettenausrichtungs-Merkmale aufweist, wobei das Verfahren außerdem Folgendes umfasst:
Bereitstellen einer äußeren Düse (18), die ein oder mehrere äußere Düsenausrichtungs-Merkmale (52) umfasst, die in das eine oder die mehreren Manschettenausrichtungs-Merkmale (56) eingreifen können,
Ausrichten des einen oder der mehreren Manschettenausrichtungs-Merkmale (56) mit dem einen oder den mehreren äußeren Düsenausrichtungs-Merkmalen (52) und Eingreifen der äußeren Düse (18) in die Manschette (50).

## Revendications

1. Ensemble de buses réglable (60) à travers lequel une lancette (20) peut être propulsée par un dispositif de crevage (10) dans une surface de crevage (21), l'ensemble de buses réglable comprenant : une buse intérieure (22) comprenant une rainure inclinée (68) et une paroi de lancette (94) ; un manchon (50) comprenant une broche à manchon (51) qui est en prise avec la rainure inclinée et coulisse par rapport à la rainure inclinée, le manchon étant adapté de manière à tourner par rapport à la buse intérieure ; et une buse extérieure (18) comprenant une surface de contact (21) qui s'étend au-delà de la paroi de lancette (94) de la buse intérieure afin d'entrer en contact avec la surface de crevage, la buse extérieure étant en prise avec le manchon et étant adapté de manière à tourner par rapport à la buse intérieure ; et dans laquelle la rainure inclinée est inclinée de manière à ce que, quand la buse extérieure tourne par rapport à la buse intérieure, la distance de laquelle la surface de contact s'étend au-delà de la paroi de lancette change d'une valeur qui correspond à l'inclinaison de la rainure inclinée.

2. Ensemble de buses selon la revendication 1, dans lequel le manchon comprend en outre un positionneur en porte-à-faux (76) et dans lequel la buse intérieure comprend en outre une pluralité d'encoches de positionnement (74) qui peuvent se mettre en prise avec le positionneur en porte-à-faux.

3. Ensemble de buses selon la revendication 1, dans lequel :
le manchon comprend en outre une rampe de manchon inclinée (70), la rampe de manchon inclinée comprenant un positionneur (76) ; et
la buse intérieure comprend en outre une rampe de buse intérieure inclinée (72), la rampe de buse intérieure inclinée comprenant une pluralité d'encoches de positionnement (74) qui peuvent se mettre en prise avec le positionneur ; et
dans lequel l'inclinaison de la rampe de manchon, l'inclinaison de la rampe de buse intérieure et l'inclinaison de la rainure inclinée sont approximativement égales.

4. Ensemble de buses selon la revendication 3, dans lequel le positionneur forme une partie en porte-à-faux de la rampe de manchon.

5. Ensemble de buses selon la revendication 3, dans lequel le positionneur forme une partie à encoches de la rampe de manchon.

6. Ensemble de buses selon la revendication 1, dans lequel la buse intérieure comprend en outre une rainure d'assemblage (66), une extrémité de la rainure d'assemblage étant à proximité d'une extrémité de la rainure inclinée, la rainure inclinée comprenant une protubérance (73) qui peut empêcher la broche de manchon de coulisser hors de la rainure inclinée dans la rainure d'assemblage.

7. Ensemble de buses selon la revendication 1, dans lequel le manchon comprend en outre une ou plusieurs fonctions d'alignement, et la buse extérieure comprend en outre une plusieurs fonctions d'alignement de buse extérieure (52) qui peuvent se mettre en prise avec la ou les fonctions d'alignement de manchon (56).

8. Ensemble de buses selon la revendication 1, dans lequel la surface de contact est concave.

9. Ensemble de buses selon la revendication 1, dans lequel la rainure inclinée comprend une rainure de surrotation.

10. Méthode d'assemblage de l'ensemble de buses réglable (60) selon la revendication 1 d'un dispositif de crevage (10), la méthode comprenant :
la fourniture d'une buse intérieure (22) comprenant une rainure d'assemblage (66) en communication avec une rainure inclinée (68), la rainure d'assemblage étant séparée de la rainure inclinée par une protubérance en saillie (73) ;
la fourniture d'un manchon (50) avec une broche de manchon (51) ; et
le rattachement du manchon à la buse intérieure en faisant coulisser la broche de manchon dans la rainure d'assemblage, sur la protubérance en saillie, et dans la rainure inclinée.

11. Méthode selon la revendication 10, dans laquelle le manchon comprend en outre un positionneur en porte-à-faux (76) et dans laquelle la buse intérieure comprend en outre une pluralité d'encoches de positionnement (74) qui peuvent se mettre en prise avec le positionneur en porte-à-faux.

12. Méthode selon la revendication 10, dans laquelle :
le manchon comprend en outre une rampe de manchon inclinée (70), la rampe de manchon inclinée comprenant un positionneur en porte-à-faux (76) ; et
la buse intérieure comprend en outre une rampe de buse intérieure inclinée (72), la rampe de buse intérieure inclinée comprenant une pluralité d'encoches de positionnement (74) qui peuvent se mettre en prise avec le positionneur en porte-à-faux ; et
dans laquelle l'inclinaison de la rampe de manchon, l'inclinaison de la rampe de buse intérieure et l'inclinaison de la rainure inclinée sont approximativement égales.

13. Le dispositif de crevage selon la revendication 12, dans lequel le positionneur forme une partie en porte-à-faux de la rampe de manchon.

14. Le dispositif de crevage selon la revendication 12, dans lequel le positionneur forme une partie à encoches de la rampe de manchon.

15. Méthode selon la revendication 10, dans laquelle la protubérance en saillie peut empêcher la broche de manchon de coulisser de la rainure inclinée dans la rainure d'assemblage.

16. Méthode selon la revendication 10, dans laquelle la rainure inclinée comprend une rainure de surrotation (75).

17. Méthode selon la revendication 10, dans laquelle le manchon comprend en outre une ou plusieurs fonctions d'alignement de manchon, la méthode comprenant en outre :
fourniture d'une buse extérieure (18) comprenant une ou plusieurs fonctions d'alignement de buse extérieure (52) qui peut se mettre en prise avec une ou plusieurs fonctions d'alignement de manchon (56) ;
l'alignement de la ou des fonctions d'alignement de manchon (56) avec la ou des fonctions d'alignement de buse extérieure (52) ; et la mise en prise de la buse extérieure (18) avec le manchon (50).
